# EUROPEAN PATENT SPECIFICATION

(11) **EP 1 251 874 B1**
(45) Date of publication and mention of the grant of the patent: **05.10.2005**
(21) Application number: 01948942.6
(22) Date of filing: 29.01.2001
(51) Int. Cl.: A61K 47/10, A61K 31/495, A61P 31/04, A61K 31/435

(54) **STABILISED PHARMACEUTICAL COMPOSITIONS AND PROCESS FOR THEIR PREPARATION COMPRISING AN ANTIBIOTIC AND AN EXPECTORANT**
STABILISIERTE PHARMAZEUTISCHE ZUSAMMENSETZUNGEN UND VERFAHREN ZU IHRER HERSTELLUNG, MIT EINEM ANTIBIOTIKUM UND EINEM EXPEKTORANS
COMPOSITIONS PHARMACEUTIQUES STABILISEES COMPRENANT UN ANTIBIOTIQUE ET UN EXPECTORANT, ET LEUR PROCEDE DE PREPARATION

(30) Priority: 31.01.2000 EP 00101857
(43) Date of publication of application: 30.10.2002
(73) Proprietor: New Pharma Research Sweden AB, 223 63 Lund (SE)
(72) Inventor: SHOA'A, Abdul Rahman, S-22 476 Lund (SE)
(74) Representative: Johansen, Marianne
(86) International application number: PCT/EP2001/000927
(87) International publication number: WO 2001/056610

(56) References cited:
- EP-A- 0 587 135
- GB-A- 2 323 532
- US-A- 4 983 586
- US-A- 5 234 683
- US-A- 5 302 486
- US-A- 5 322 777
- SCHMIDT ET AL: "Treatment of Acute Respiratory Tract Diseases in Cattle with Bisolvon in Combination with either Enrofloxacin, Cefquinome, Ceftiofur or Florfenicol" TIERARZTLICHES PRAXIS, AUSGABE G, GROSSTIERE NUTZTIERE, vol. 26, no. 3, May 1998 (1998-05), pages 127-132, XP000911130 cited in the application
- TSUDA ET AL: "Toxic Pustuloderma Induced by Ofloxacin" ACTA DERMATO-VENEREOLOGICA, vol. 73, no. 5, October 1993 (1993-10), pages 382-384, XP000911174
- HIBBERT: "Abdominal Distension in a Cat" THE VETERINARY RECORD, vol. 141, no. 2, 12 July 1997 (1997-07-12), page 56 XP000911173

## Description

### Field of the invention:

The present invention concerns aqueous compositions comprising an antibiotic, the compositions being intended to be used in the pharmaceutical field, and preferably for the prophylaxis or treatment of bacterial diseases affecting humans or farm and domestic animals. The present invention concerns also a process for the preparation of the compositions.

### Background of the invention:

It is known that quinolone- or naphthyridone-type antibiotics are useful as agents for the treatment of bacterial diseases affecting humans or animals. Antibiotics belonging to this group are for example enrofloxacin, ciprofloxacin, ofloxacin, flumequine, norfloxacin, nalidixic acid, cinoxacin, clinafloxacin, difloxacin, enoxacin, fleroxacin, miloxacin, nadifloxacin, pefloxacin, pipemidic acid, rosoxacin, rufloxacin, sparfloxacin, temafloxacin, tosufloxacin, trovafloxacin, orbifloxacin, marbofloxacin, benofloxacin, binfloxacin, danofloxacin, sarafloxacin, premafloxacin or ibafloxacin.

An injectable solution of enrofloxacin is sold, for example, by the company Bayer AG, Germany, under the trade name Baytril®.

Enrofloxacin is the generic name of a fluoroquinolone carboxylic compound. More specifically, enrofloxacin is 1-cyclopropyl-7-(4-ethyl-1-piperazinyl)-6-fluoro-1,4-dihydro-4-oxo-3-quinoline carboxylic acid. Enrofloxacin has a tendency to precipitate in solution.

Suitable salts of quinolones are those of inorganic or organic salts selected from hydrochloric acid, hydrobromic acid, hydroiodic acid, sulfuric acid, phosphoric acid, acetic acid, succinic acid, phosphonic acid, malic acid, sodium hydroxyde, potassium hydroxyde, aluminium hydroxyde, piperidine, morpholine, ethylamine and triethylamine.

Several studies have been conducted to improve the efficacy of the antibiotic treatment. The following examples show in which direction the studies were conducted.

Bonse et al. (US Patent 5,998,418) describes injection and infusion aqueous solutions based on the salt of enrofloxacin with specific polycarboxylic acids or amino-acids, for combating bacteria in an animal. The pH of the aqueous solution is 3 to 5.5. According to the author, the advantage of these solutions is that the active substance is present in high levels in the blood only a short time after administration and that it is eliminated rapidly.

Also, Copeland et al. (US Patent 5,756,506) describes a process for the treatment of bacterial infections like the bovine respiratory disease or the swine pleuropneumonia, by administering by injection a composition containing a single high dose of between 7.5 and 15 mg/kg of enrofloxacin to the animal. According to the author, the advantage is that the single high dose treatment can replace repeated treatments.

In another reference, Vetter et al. (US Patent 5,808,076) describes orally administrable formulations containing a quinolone- or naphthyridone-carboxylic acid embonate and an excipient, like for example corn starch, colloidal silica, vaseline or paraffin (used as a carrier). According to the author, the presence of embonic acid in the formulation has the advantage of masking the bitter taste of the quinolone-or naphthyridone-carboxylic acid and therefore improves the acceptance of the orally administrable formulation by the subject animal.

From the above examples, it can be concluded that to improve the efficacy of the antibiotic treatment, either the authors have tried to change the posology (changing the dose of antibiotic injected), or they have tried to improve the absorption or the acceptance of the substance by the subject animal. In the latter case, further compounds were added in the composition.

Other studies to improve the effect of the antibiotic treatment have been made, such as for example by combination therapy (a treatment whereby several agents are administered separately but simultaneously to the patient).

Schmidt et al. (Tierärtzl. Prax. 1998, 26(G), pages 127-132) describes such a combination therapy with enrofloxacin and with a bronchosecretolytic agent, bromhexine-HCl.

Bromhexine-HCl is the generic name of N-(2-amino-3,5-dibromobenzyl)-N-methyl-cyclohexylamine hydrochloride and is known as an anti-histamine, expectorant, mucolytic drug. It is sold, for example, by the company Boehringer Ingelheim Vetmedia GmbH, Germany, under the trade name Bisolvon®, in a dry form or as a 0.2% solution. Bromhexine-HCl is very insoluble in water and is known to precipitate in solutions of pH greater than six.

The aim of the clinical studies of Schmidt et al. was to determine the clinical efficacy of a combined treatment with enrofloxacin and bromhexine-HCl in respiratory diseased bovines, whereby during the treatment bromhexine-HCl was in a first stage injected parenterally and in a second stage given orally. According to the authors, an acceleration of the recovery of the animals treated by the combination therapy was observed when compared to the treatment with the antibiotic alone.

In other respects, it is known from the literature (see Kern et al., US Patent 5,808,076) to combine bromhexine-HCl and erythromycin, erythromycin lactobionate, tylosin, oxytetracycline-HCl, ampicillin or benzathine ampicillin, to improve the absorption of the antibacterial substance. Other antibiotics are cited in this reference which could, according to the author, also be combined either with bromhexine-HCl or with another benzylamine derivative, ambroxol. These antibiotics belong to the tetracycline, betalactam, erythromycin, spiramycin, tylosin, oleandomycin, chloramphenicol, thiamphenicol or sulfonamide groups.

However, the prior art does not disclose a composition which combines both an antibiotic of the quinolone- or naphthyridone-type, like enrofloxacin, and bromhexine-HCl.

Studies made by the present inventor have shown that bromhexine-HCl cannot be solubilised in the usual antibiotic compositions containing a quinolone- or naphthyridone-type antibiotic like enrofloxacin. Bromhexine-HCl even enhances the tendency of enrofloxacin to precipitate in the composition.

Surprisingly, the inventor of the present invention has found that an aqueous stable composition comprising as essential active ingredients enrofloxacin and bromhexine-HCl is achieved when glacial acetic acid and a stabilising agent is added to the composition, the stabilising agent being preferably selected from the combination of a non-ionic surfactant and of benzyl alcohol, N-methylpyrrolidone, 2-pyrrolidone, dimethylacetamate (DMA), dimethylformamide (DMF), dimethylsulfoxide (DMSO), taken alone or in combination. The non-ionic surfactant is essential to keep the composition stable.

A stable composition in the meaning of the present invention is a composition wherein the compounds are present in a soluble form, i.e. there is no precipitate, the composition remaining stable during storage at ambient temperature for at least two years.

Hence, the composition can be used in countries where the possibility of keeping it in a stable temperature environment is not guaranteed.

The inventor of the present invention has also found that the same effect is obtained when enrofloxacin is replaced by any other quinolone- or naphthyridone-type antibiotic or derivative thereof.

The inventor of the present invention has also found that the same effect is obtained when bromhexine-HCl is replaced by any other expectorant or its salts, like for example ambroxol or its salts, carbocysteine, guaiacol or its benzoate or phosphate salts, or guaiacolsulfonate or its salts.

The inventor of the present invention has also found a process for the preparation of said composition.

The inventor of the present invention has also found that said composition is effective for the prophylaxis or treatment of diseases in humans and animals, in particular the treatment of bacterial infections due to microorganisms pathogenic to humans and animals.

Thus, the main object of the present invention is to provide new compositions which are effective as antibacterial agents.

Another object of the present invention is to provide these compositions as compositions which are stable during storage over a wide range of temperature.

Another object of the present invention is to provide these compositions as compositions which can be administered orally or by injection.

Another object of the present invention is to provide a process for the preparation of these compositions.

One of the advantages of the present invention is therefore that the composition can be stored in a stable manner in the form in which it will be administered.

Another advantage of the present invention is that the composition has better absorption characteristics than a composition which contains only the antibiotic.

Further problems which can be solved by this invention with respect to known prior art compositions will become apparent to the reader of the following description.

### Summary of the invention:

The present invention provides a solution to the aforementioned problems.

The aforementioned object is achieved by a composition having the features defined in claim 1 and comprising as essential ingredients a quinolone- or naphthyridone-type antibiotic or derivative thereof, an expectorant, glacial acetic acid, water and a stabilising agent, the stabilising agent being selected from the combination of a non-ionic surfactant with benzyl alcohol, N-methylpyrrolidone, 2-pyrrolidone, dimethylacetamate (DMA), dimethylformamide (DMF), dimethylsulfoxide (DMSO), taken alone or in combination.

The aforementioned object is also achieved by the use of the compositions for the prophylaxis or treatment of diseases, and most preferably bacterial infections affecting humans or animals.

Preferred embodiments of the invention, including the use of chemical derivatives of the essential compounds of the composition or preferred concentrations, the addition of optional ingredients in the composition like for example formalin, excipients, preservatives, vitamins, further stabilising agents, carriers, antioxidants, photostabilizers, colorants, other absorption-promoting substances, or the use of the composition for the treatment of specific bacterial diseases in humans or animal are defined in the dependent claims.

The aforementioned object is also achieved by the process for the preparation of the compositions comprising: (i) in a first stage the preparation of solution A comprising the dispersion of the expectorant in part of the glacial acetic acid, the addition of the stabilising agent(s) followed by the addition of part of the water, and the mixing and heating until the expectorant is completely dissolved; (ii) in a second stage the preparation of solution B comprising the dissolution of the quinolone- or naphthyridone-type antibiotic or derivative thereof in the rest of the glacial acetic acid and water by simple mixing at room temperature; (iii) in a third stage the mixing of A and B and homogenisation, followed by the filtration of the composition through a 60 micron filter, followed by filtration through a 6 micron filter. In the case of a formulation intended to be injected, further filtration through a 0.1 micron filter is carried out.

The aforementioned object is also achieved by an alternative process for the preparation of the compositions wherein the expectorant is first dissolved in the whole amount of glacial acetic acid, followed by the addition of the stabilising agent(s), the addition of water, the mixing and heating until the expectorant is completely dissolved, and then the addition of the quinolone- or naphthyridone-type antibiotic or derivative thereof and mixing until dissolution, followed by the same filtration procedure as above, with the proviso that the non-ionic surfactant is added only after the antibiotic.

Unless otherwise specified, the percentages of ingredients used in the compositions are defined as weight per volume. The preferred concentrations or ranges of concentrations mentioned hereafter are concentrations which are more suitable to achieve the object of the present invention.

### Description of preferred embodiments:

The compositions according to the present invention comprise as essential ingredients a quinolone- or naphthyridone-type antibiotic or an active chemical derivative thereof, an expectorant, glacial acetic acid, water and a stabilising agent.

The quinolone- or naphthyridone-type antibiotic is preferably selected from enrofloxacin, ciprofloxacin, ofloxacin, flumequine, norfloxacin, nalidixic acid, cinoxacin, clinafloxacin, difloxacin, enoxacin, fleroxacin, miloxacin, nadifloxacin, pefloxacin, pipemidic acid, rosoxacin, rufloxacin, sparfloxacin, temafloxacin, tosufloxacin, trovafloxacin, orbifloxacin, marbofloxacin, benofloxacin, binfloxacin, danofloxacin, sarafloxacin, premafloxacin or ibafloxacin. Enrofloxacin, ciprofloxacin, norfloxacin, danofloxacin or sarafloxacin will preferably be used.

The amount of quinolone- or naphthyridone-type antibiotic or its active chemical derivative in the composition is preferably between 5 and 40% weight/volume. Most preferably, the composition contains 20% weight/volume of quinolone- or naphthyridone-type antibiotic or its active chemical derivative.

The expectorant is preferably selected from bromhexine-HCL, ambroxol (4[[(2-amino3,5-dibromophenyl)methyl]amino]cyclohexanol) or its salts, carbocysteine (carboxymethylcysteine), guaiacol (2-methoxyphenol) or its benzoate or phosphate salts, guaifenesin (3-(2-methoxyphenoxy)-1,2-propanediol), potassium guaiacolsulfonate (a mixture of potassium salts of 4- and 5- guaiacolsulfonic acid, also known as sulfoguaiacol, thiosol or orthosol). Bromhexine-HCl will preferably be used.

The amount of the expectorant in the composition is preferably between 0.1 and 4% weight/volume. Most preferably, the composition contains 1% weight/volume of expectorant.

The amount of glacial acetic acid in the composition is preferably between 5 and 16% weight/volume. Most preferably, the composition contains 8% weight/volume of glacial acetic acid.

The amount of water in the composition is preferably superior or equal to 35% weight/volume.

The stabilising agent is a combination of a non-ionic surfactant with benzyl alcohol, N-methylpyrrolidone, 2-pyrrolidone, dimethylacetamate (DMA), dimethylformamide (DMF), dimethylsulfoxide (DMSO), taken alone or in combination.

The non-ionic surfactant is preferably selected from the group consisting of the non-ionic surfactants having an HLB (Hydrophilic Lipophilic Balance) of between 10 and 18. Examples of such surfactants are: esters of polyhydric alcohols such as glycol and glycerol esters, macrogol esters such as polyoxyl 40 stearate, polyoxyl 50 stearate and polyoxyl 40 hydrogenated castor oil, sorbitan esters such as sorbitan monopalmitate and sorbitan monostearate, polysorbates such as Tween-80 and polysorbate 60, macrogol ethers such as cetomacrogol 1000, polyoxyl 10 oleyl ether and polyoxyl 20 cetostearyl ether, long-chain alcohols such as cetostearyl alcohol and cetyl alcohol, poloxamers such as poloxamer 188, and polyvinylalcohols. The non-ionic surfactant can also be a mixture of the above-mentioned non-ionic surfactants.

Preferably, the stabilising agent is a combination of Tween-80 and 2-pyrrolidone, Tween-80 and N-methylpyrrolidone, Tween-80 and dimethylacetamate (DMA), Tween-80 and dimethylformamide (DMF), Tween-80 and dimethylsulfoxide (DMSO), or Tween-80, 2-pyrrolidone and benzyl alcohol.

The total amount of stabilising agent in the composition is preferably between 9 and 30% weight/volume.

The amount of Tween-80 in the composition is preferably between 5 and 20% weight/volume. Most preferably, 10% weight/volume of Tween-80 will be used.

The amount of N-methylpyrrolidone, 2-pyrrolidone, dimethylacetamate (DMA), dimethylformamide (DMF) or dimethylsulfoxide (DMSO) in the composition is preferably between 2 and 20% weight/volume. Most preferably, 4% weight/volume of one of these agents will be used.

The amount of benzyl alcohol in the composition is preferably between 0.1 and 5% weight/volume.

Variations of the amount of stabilising agent depend on the combination used and on the amount of antibiotic and expectorant in the composition.

Preferably, when the composition comprises 20% weight/volume of enrofloxacin and 1% weight/volume of bromhexine-HCl, a combination of 4% weight/volume of 2-pyrrolidone and 10% weight/volume of Tween-80 will be used as stabilising agent.

Optionally, formalin may be added in the compositions intended to be used for oral administration, since the inventor of the present invention has discovered that an addition of formalin can further enhance and broaden the antimicrobial activity of the composition. The amount of formalin added in the composition is preferably between 0.1 and 3% weight/volume.

Formalin is the name of a 37% (w/w) aqueous solution of formaldehyde. This solution, which can be purchased from various sources, contains usually between less than 1% to 15% of methanol and less than 0.05% (w/w) of formic acid.

The compositions may also comprise optionally other agents like excipients, preservatives, vitamins, further stabilizing agents, carriers, antioxidants, photostabilizers, colorants, other absorption-promoting substances, thickeners or any other agent or additive commonly used in veterinary or medical compositions which is for example useful for the stability of the composition or permits to improve the formulation for a specific way of administration without altering the antibacterial activity.

When necessary, the composition is generally complemented to the required final volume by the addition of deionized water.

The process for the preparation of the composition comprises:
(i) in a first stage the preparation of a solution A comprising the dispersion of the expectorant in part of the glacial acetic acid, the addition of the stabilising agent(s) followed by the addition of part of the water, and the mixing and heating until the expectorant is completely dissolved;
(ii) in a second stage the preparation of a solution B comprising the dissolution of the quinolone- or naphthyridone-type antibiotic or derivative thereof in the rest of the glacial acetic acid and water by simple mixing at room temperature; (iii) in a third stage the mixing of A and B and the homogenisation of the obtained composition, followed by the filtration of the composition through a 60 micron filter, followed by filtration through a 6 micron filter. In the case of a formulation intended to be injected, further filtration through a 0.1 micron filter is carried out to ensure that no particles of a size higher than 0.1 micron, like e.g. microorganisms, are present in the composition.

This process is most suitably used when the expectorant is bromhexine-HCl.

The parts of the glacial acetic acid and water used in solution A represent preferably about 40% of the total amount of each of these components present in the final composition.

An alternative process for the preparation of the composition is possible, wherein the expectorant is first dissolved in the whole amount of glacial acetic acid, followed by the addition of the stabilising agent(s), the addition of water, the mixing and heating until the expectorant is completely dissolved, and then the addition of the quinolone- or naphthyridone-type antibiotic or derivative thereof and mixing until dissolution, followed by the same filtration procedure as above, with the proviso that the non-ionic surfactant is added only after the antibiotic in order to avoid an increase of the viscosity which would lead to a delay in the solubilisation of the antibiotic.

This process is most suitably used when the expectorant is bromhexine-HCl.

In a preferred embodiment applicable to both alternate processes, the temperature up to which the solution is heated until the expectorant is completely dissolved is between about +50°C and +60°C. Most preferably a heating up to +60°C is carried out.

The optional agents like formalin will preferably be added at the end of the process, but before filtration.

Generally, the compositions are intended to be administered orally or by injection and will be prepared in a suitable form. However, it should be noted that the form in which the compositions are administered depends upon the particular bacterial infection to be treated and may be adapted in order, for example, to deliver the agent closer to the site of infection. Thus, in the case of infections of a topical nature, a formulation for topical application may be prepared and topical application may be used.

The compositions are effective for the prophylaxis or treatment of diseases in humans and animals, in particular the treatment of bacterial infections due to microorganisms pathogenic to humans and animals.

The microorganisms include, for example, microorganisms of the following types: streptococci, staphylococci, pseudomonas, enterobacteria as e.g. Escherichia coli or salmonella, listeria, clostridium, corynebacteria, mycobacteria, actinomycetes, rickettsia, chlamydia or mycoplasma.

The posology and way of administration depend on the subject to be treated and the stage and severity of the infection.

For example, for systemic treatment of cattle and calves suffering from pneumoniae, the composition is administered by intramuscular injection and the antibiotic is given at a daily dose of about 2.5 mg/kg body weight, one time each day and for generally between two and five successive days.

For systemic treatment of poultry infected by Escherichia coli, the composition is given orally, at a dose of 1 ml of composition per 4 litre of drinking water, and continuously for three consecutive days. This corresponds approximately to an antibiotic dose of 10 mg/kg body weight in the case of chicken.

The invention will now be described in more detail with reference to the following examples.

### Examples:

The amounts of ingredients are given in % of weight/volume. The volume is adjusted by addition of deionized water.

### Example 1:

A composition comprising:

| | |
|---|---|
| Enrofloxacin | 20% |
| Bromhexine-HCl | 1% |
| Acetic acid | 8% |
| Tween-80 | 10% |
| 2-pyrrolidone | 4% |
| Water | q.s.p. 100% |

### Example 2:

A process for the preparation of 100 ml of the composition of example 1 wherein:
(i) in a first stage a solution A is prepared, comprising the dispersion of 1 g of bromhexine-HCl in 3 g of glacial acetic acid, the addition of 4 g of 2-pyrrolidone and 10 g of Tween-80, followed by the addition of 22 g of water, and the mixing and heating up to + 60°C until bromhexine-HCl is completely dissolved;
(ii) in a second stage a solution B is prepared, comprising the dissolution of 20 g enrofloxacin in 5 g of glacial acetic acid and 35 g of water by simple mixing at room temperature;
(iii) in a third stage A and B are mixed together and homogenised, and the composition is filtrated through a 60 micron filter, followed by filtration through a 6 micron filter.

In the case of a formulation intended to be injected, further filtration through a 0.1 micron filter is carried out.

### Example 3:

A process for the preparation of 100 ml of the composition of example 1 wherein:
1 g of bromhexine-HCl is dissolved in 8 g of glacial acetic acid, followed by the addition of 4 g of 2-pyrrolidone, the addition of 57 g of water, the mixing and heating up to +60°C until bromhexine-HCl is completely dissolved, and then the addition of 20 g of enrofloxacin and mixing until dissolution, followed by the addition of 10 g of Tween-80, and followed by the same filtration procedure as in Example

### Example 4:

A composition comprising:

| | |
|---|---|
| Enrofloxacin | 20% |
| Bromhexine-HCl | 1% |
| Acetic acid | 8% |
| Tween-80 | 5% |
| 2-pyrrolidone | 4% |
| Water | q.s.p. 100% |

### Example 5:

A composition comprising:

| | |
|---|---|
| Enrofloxacin | 5% |
| Bromhexine-HCl | 1% |
| Acetic acid | 5% |
| Tween-80 | 7.5% |
| 2-pyrrolidone | 4% |
| Water | q.s.p. 100% |

### Example 6:

A composition comprising:

| | |
|---|---|
| Enrofloxacin | 10% |
| Bromhexine-HCl | 0.5% |
| Acetic acid | 8% |
| Cremophore | 10% |
| 2-pyrrolidone | 4% |
| Water | q.s.p. 100% |

### Example 7:

A composition comprising:

| | |
|---|---|
| Enrofloxacin | 20% |
| Bromhexine-HCl | 1% |
| Acetic acid | 8% |
| Tween-80 | 8% |
| DMA | 4% |
| Water | q.s.p. 100% |

### Example 8:

A composition comprising:

| | |
|---|---|
| Enrofloxacin | 20% |
| Bromhexine-HCl | 1% |
| Acetic acid | 8% |
| Tween-80 | 10% |
| DMF | 4% |
| Water | q.s.p. 100% |

### Example 9:

A composition comprising:

| | |
|---|---|
| Enrofloxacin | 20% |
| Bromhexine-HCl | 1% |
| Acetic acid | 8% |
| Tween-80 | 10% |
| DMSO | 4% |
| Water | q.s.p. 100% |

### Example 10:

A composition comprising:

| | |
|---|---|
| Enrofloxacin | 20% |
| Bromhexine-HCl | 1% |
| Acetic acid | 8% |
| Tween-80 | 10% |
| 2-pyrrolidone | 4% |
| Benzyl alcohol | 1% |
| Water | q.s.p. 100% |

### Example 11:

A composition comprising:

| | |
|---|---|
| Enrofloxacin | 30% |
| Bromhexine-HCl | 1.5% |
| Acetic acid | 15% |
| Tween-80 | 20% |
| 2-pyrrolidone | 4% |
| Water | q.s.p. 100% |

### Example 12:

A composition comprising:

| | |
|---|---|
| Enrofloxacin | 20% |
| Bromhexine-HCl | 1% |
| Acetic acid | 8% |
| Tween-80 | 10% |
| 2-pyrrolidone | 4% |
| Formalin (37%) | 3% |
| Water | q.s.p. 100% |

Accelerated stability studies in accordance with CVM Guidelines 5 show that all the above-exemplified compositions have a shelf or more than two years.

A composition like the composition of example 1 can remain stable formore than one year in the refrigerator. For comparison, already known formulations of enrofloxacin of the prior art, like for exame the formulation of Baytril®, has a tendency to precipitate within a few days is kept in the refrigerator.

When used for the prophylaxis or treatment of bacterial diseases in humans and animals, the compositions in accordance with the present invention are more effective than compositions which do not contain the combination of the antibiotic and bromhexine-HCl.

The following examples illustrate the efficacy of the compositions of the present invention and their industrial applicability.

### Evaluation of the efficacy of a composition in accordance with the present invention in the treatment of Escherichia coli infected chickens.

The aim of this study was to evaluate the effect of a treatment with a composition in accordance with the present invention on the mortality and clinical signs of chickens naturally infected by Escherichia coli, and to compare the efficacy of this treatment with the efficacy of a treatment with a composition containing only the antibiotic.

Thus, the following experiment was carried out.

10 000 broiler chickens aged 1 day were used for the experiment. The birds were infected by natural infection by Escherichia coli.

The birds were then divided into 2 equal groups of 5 000 birds each. The first group was treated with a composition containing only enrofloxacin. The second group was treated with the composition of Example 1 of the present invention, containing both enrofloxacin and bromhexine-HCl. Thus, 1 ml of composition contained either 200 mg of enrofloxacin (composition for the treatment of the first group) or 200 mg of enrofloxacin and 10 mg of bromhexine-HCl (composition for the treatment of the second group).

For both groups, the treatment was made orally, for three consecutive days, and using the drinking water, at a dose of 1 ml of active composition per 4 litres of drinking water.

To assess the efficacy of the treatment, the mortality and body weight of the animals were recorded for each group over a period of ten days following the treatment. The results are summarised in the following Table I.

**Table I**

| Treatment | Mortality | | Weight | |
|---|---|---|---|---|
| | dead birds | % | g/bird | % |
| Enrofloxacin | 113/5000 | 2.23 | 1810 | -- |
| Enrofloxacin + bromhexine-HCl | 32/5000 | 0.6 | 1950 | 7.7 |

As is clear from Table I, the combined treatment with enrofloxacin and bromhexine-HCl is more effective in controlling the mortality associated with the bacterial infection than the treatment using only enrofloxacin. Furthermore, the group of birds treated with the combination of enrofloxacin and bromhexine-HCl showed a significant increase in body weight.

Therefore, the composition in accordance with the present invention has clearly a therapeutic effect on chickens infected by Escherichia coli.

### Evaluation of the efficacy of a composition in accordance with the present invention in the treatment of calves suffering from a pneumonia infection

The aim of this study was to evaluate the effect of a treatment with a composition in accordance with the present invention on the mortality and clinical signs of calves suffering from a pneumonia infection, and to compare the efficacy of this treatment with the efficacy of a treatment with a composition containing only the antibiotic.

Thus, the following experiment was carried out.

20 calves aged 1 to 4 months were used for the experiment. The animals were suffering from a pneumonia infection, characterised by severe respiratory disorders including coughing, rale and increased body temperature (measured between +39.5°C and +41°C).

The calves were divided into 2 equal groups of 10 animals each. The first group was treated with a composition containing only enrofloxacin. The second group was treated with the composition of Example 1 of the present invention, i.e. containing both enrofloxacin and bromhexine-HCl. Thus, 1 ml of composition contained either 200 mg of enrofloxacin (composition for the treatment of the first group) or 200 mg of enrofloxacin and 10 mg of bromhexine-HCl (composition for the treatment of the second group).

For both groups, the treatment was made by intramuscular injection, once a day and for four consecutive days, at a dose of 1 ml of active composition per 80 kg of animal body weight.

To assess the efficacy of the treatment, the mortality and body temperature of the animals were recorded for each group over a period of ten days following the treatment. The results after 5 days are summarised in the following Table II.

**Table II**

| Treatment | Mortality | | Body temperature |
|---|---|---|---|
| | dead animals | % | |
| Enrofloxacin | 1/10 | 10 | over 41.5°C |
| Enrofloxacin + bromhexine-HCl | 0/10 | 0 | returned to normal |

As is clear from Table II, the combined treatment with enrofloxacin and bromhexine-HCl is more effective in curing the disease associated with the infection than the treatment using only enrofloxacin, since after 5 days the body temperature of the animals treated with enrofloxacin and bromhexine-HCl returned to a normal value, whereas almost no change was observed for the animals which were treated with only the antibiotic. Furthermore, no animal died in the group treated with the combination of enrofloxacin and bromhexine-HCl.

Therefore, the composition in accordance with the present invention has clearly a therapeutic effect on calves suffering from an infection like pneumonia.

### Evaluation of the absorption-promoting effect of bromhexine-HCl on enrofloxacin in the compositions in accordance with the present invention

Experiments were made to measure the pharmaco-kinetic parameters of the antibacterial activity in the blood of animals treated with either a composition in accordance with the present invention or a composition containing only the antibiotic.

The aim of this study was to evaluate the absorption-promoting effect of bromhexine-HCl on enrofloxacin. The experiments were performed on chickens, sheep, goats and cattle, and the treatment was made either orally in the case of chickens or by intramuscular injection in the case of the other animals.

In all cases, the results showed a significant increase of the blood level of enrofloxacin when the antibiotic was given in combination with bromhexine-HCl. The absorption half-life of enrofloxacin was also shorter when the antibiotic was given in combination with bromhexine-HCl. These results demonstrate clearly an absorption-promoting effect of bromhexine-HCl on enrofloxacin.

The compositions according to the present invention can therefore be used as prophylaxis or treatment for animals or humans infected by bacterial diseases.

Specifically, other compounds like chemical derivatives of the active compounds cited herein could be used, as soon as the modification does not lead to a substantial loss of the activity of the compound. For example, derivatives of the quinolone- or naphthyridone-type antibiotics are their C1-C4 alkyl esters.

## Claims

1. An aqueous single formulation composition comprising a quinolone- or naphthyridone-type antibiotic, or an active chemical derivative thereof selected from the C₁-C₄ ester derivatives, an expectorant, a stabilising agent, glacial acetic acid and water,
the stabilising agent being selected from the combination of a non-ionic surfactant with one or more compounds selected from benzyl alcohol, N-methylpyrrolidone, 2-pyrrolidone, dimethylacetamate (DMA), dimethylformamide (DMF) and dimethylsulfoxide (DMSO),
the non-ionic surfactant having an HLB of between 10 and 18 and being selected from the group consisting of esters of polyhydric alcohols, macrogol esters, sorbitan esters, polysorbates, macrogol ethers, poloxamers and polyvinylalcohols, or a mixture thereof.

2. A composition according to claim 1 wherein the non-ionic surfactant is Tween-80 or Cremophore.

3. A composition according to claim 1 or 2 wherein the expectorant is selected from bromhexine-HCL, ambroxol or its salts, carbocysteine, guaiacol or its benzoate or phosphate salts, guaifenesin, or potassium guaiacolsulfonate.

4. A composition according to claim 3 wherein the expectorant is bromhexine-HCl.

5. A composition according to any one of claims 1 to 4 wherein the antibiotic is selected from enrofloxacin, ciprofloxacin, norfloxacin, danofloxacin or sarafloxacin.

6. A composition according to any one of claims 1 to 5 wherein the amount of antibiotic is between 5% and 40% weight/volume.

7. A composition according to claim 6 wherein the amount of antibiotic is 20% weight/volume.

8. A composition according to any one of claims 1 to 7 wherein the amount of expectorant is between 0.1% and 4% weight/volume.

9. A composition according to claim 8 wherein the amount of expectorant is 1% weight/volume.

10. A composition according to any one of claims 1 to 9 wherein the amount of acetic acid is between 5% and 16% weight/volume.

11. A composition according to claim 10 wherein the amount of acetic acid is 8% weight/volume.

12. A composition according to any one of claims 1 to 11 wherein the amount of water is superior or equal to 35% weight/volume.

13. A composition according to any one of claims 1 to 12 wherein the stabilising agent is a combination selected from the group consisting of Tween-80 and 2-pyrrolidone, Tween-80 and N-methylpyrrolidone, Tween-80 and dimethylacetamate (DMA), Tween-80 and dimethylformamide (DMF), Tween-80 and dimethylsulfoxide (DMSO), or Tween-80, 2-pyrrolidone and benzyl alcohol.

14. A composition according to any one of claims 1 to 13 wherein the amount of stabilising agent is between 9% and 30% weight/volume.

15. A composition according to any one of claims 1 to 14 which comprises 20% weight/volume of enrofloxacin, 1% weight/volume of bromhexine-HCl, 4% weight/volume of 2-pyrrolidone and 10% weight/volume of Tween-80.

16. A composition according to any one of claims 1 to 15 which further comprises formalin.

17. A composition according to claim 16 wherein the amount of formalin is between 0.1 and 3% weight/volume.

18. A composition according to any one of claims 1 to 17 which further comprises excipients, preservatives, vitamins, carriers, antioxidants, photostabilizers, colorants, or thickeners.

19. A composition according to any one of claims 1 to 18 for its use as a medicament.

20. Use of a composition according to any one of claims 1 to 19 for the preparation of a medicament for the prophylactic or therapeutic treatment of bacterial diseases.

21. Process for the preparation of a composition according to any one of claims 1 to 15 comprising: (i) in a first stage the preparation of a solution A comprising the dispersion of the expectorant in part of the glacial acetic acid, the addition of the stabilising agent(s) followed by the addition of part of the water, and the mixing and heating until the expectorant is completely dissolved; (ii) in a second stage the preparation of a solution B comprising the dissolution of the quinolone- or naphthyridone-type antibiotic or derivative thereof in the rest of the glacial acetic acid and water by simple mixing at room temperature; (iii) in a third stage the mixing of A and B and the homogenisation of the obtained composition.

22. Process according to claim 21 wherein the part of the glacial acetic acid and water used in solution A represents about 40% of the total amount of each of these components present in the final composition.

23. Process for the preparation of a composition according to any one of claims 1 to 15 comprising the steps of first dissolving the expectorant in the whole amount of glacial acetic acid, followed by the addition of the stabilising agent(s), the addition of water, the mixing and heating until the expectorant is completely dissolved, and then the addition of the quinolone- or naphthyridone-type antibiotic or derivative thereof and mixing until dissolution, with the proviso that the non-ionic surfactant is added only after the antibiotic.

24. Process according to any one of claims 20 to 23 wherein the temperature up to which the solution is heated until the expectorant is completely dissolved is between about +50°C and +60°C.

25. Process according to claim 24 wherein the temperature up to which the solution is heated until the expectorant is completely dissolved is +60°C.

26. Process according to any one of claims 20 to 25 further comprising a step of filtration of the composition.

## Patentansprüche

1. Wässrige Zusammensetzung, die in einer einzigen Zubereitung ein Antibiotikum des Chinolon- oder Naphthyridon-Typs oder ein aktives chemisches Derivat davon, das aus den C₁-C₄-Esterderivaten ausgewählt ist, ein Expectorans, einen Stabilisator, Eisessig und Wasser umfasst;
wobei der Stabilisator ausgewählt ist aus der Kombination eines nichtionischen Tensids mit einer oder mehreren Verbindungen, die aus Benzylalkohol, N-Methylpyrrolidon, 2-Pyrrolidon, Dimethylacetamat (DMA), Dimethylformamid (DMF) und Dimethylsulfoxid (DMSO) ausgewählt sind;
wobei das nichtionische Tensid einen HLB-Wert zwischen 10 und 18 hat und aus der Gruppe, die aus Estern von mehrwertigen Alkoholen, Makrogolestern, Sorbitanestern, Polysorbaten, Makrogolethern, Poloxameren und Polyvinylalkoholen besteht, oder einem Gemisch davon ausgewählt ist.

2. Zusammensetzung gemäß Anspruch 1, wobei es sich bei dem nichtionischen Tensid um Tween-80 oder Cremophore handelt.

3. Zusammensetzung gemäß Anspruch 1 oder 2, wobei das Expectorans aus Bromhexin-HCl, Ambroxol oder seinen Salzen, Carbocystein, Guaiacol oder seinen Benzoat- oder Phosphatsalzen, Guaifenesin oder Kaliumguaiacolsulfonat ausgewählt ist.

4. Zusammensetzung gemäß Anspruch 3, wobei es sich bei dem Expectorans um Bromhexin-HCl handelt.

5. Zusammensetzung gemäß einem der Ansprüche 1 bis 4, wobei das Antibiotikum aus Enrofloxacin, Ciprofloxacin, Norfloxacin, Danofloxacin oder Sarafloxacin ausgewählt ist.

6. Zusammensetzung gemäß einem der Ansprüche 1 bis 5, wobei die Menge des Antibiotikums zwischen 5% und 40% (w/v) liegt.

7. Zusammensetzung gemäß Anspruch 6, wobei die Menge des Antibiotikums 20% (w/v) beträgt.

8. Zusammensetzung gemäß einem der Ansprüche 1 bis 7, wobei die Menge des Expectorans zwischen 0,1% und 4% (w/v) liegt.

9. Zusammensetzung gemäß Anspruch 8, wobei die Menge des Expectorans 1% (w/v) beträgt.

10. Zusammensetzung gemäß einem der Ansprüche 1 bis 9, wobei die Menge der Essigsäure zwischen 5% und 16% (w/v) liegt.

11. Zusammensetzung gemäß Anspruch 10, wobei die Menge der Essigsäure 8% (w/v) beträgt.

12. Zusammensetzung gemäß einem der Ansprüche 1 bis 11, wobei die Menge des Wassers größer oder gleich 35% (w/v) ist.

13. Zusammensetzung gemäß einem der Ansprüche 1 bis 12, wobei der Stabilisator eine Kombination ist, die aus der Gruppe ausgewählt ist, die aus Tween-80 und 2-Pyrrolidon, Tween-80 und N-Methylpyrrolidon, Tween-80 und Dimethylacetamat (DMA), Tween-80 und Dimethylformamid (DMF), Tween-80 und Dimethylsulfoxid (DMSO) oder Tween-80, 2-Pyrrolidon und Benzylalkohol besteht.

14. Zusammensetzung gemäß einem der Ansprüche 1 bis 13, wobei die Menge des Stabilisators zwischen 9% und 30% (w/v) liegt.

15. Zusammensetzung gemäß einem der Ansprüche 1 bis 14, die 20% (w/v) Enrofloxacin, 1% (w/v) Bromhexin-HCl, 4% (w/v) 2-Pyrrolidon und 10% (w/v) Tween-80 umfasst.

16. Zusammensetzung gemäß einem der Ansprüche 1 bis 15, die weiterhin Formalin umfasst.

17. Zusammensetzung gemäß Anspruch 16, wobei die Menge des Formalins zwischen 0,1 und 3% (w/v) liegt.

18. Zusammensetzung gemäß einem der Ansprüche 1 bis 17, die weiterhin Arzneimittelhilfsstoffe, Konservierungsstoffe, Vitamine, Träger, Antioxidantien, Photostabilisatoren, Färbemittel oder Verdickungsmittel umfasst.

19. Zusammensetzung gemäß einem der Ansprüche 1 bis 18 zur Verwendung als Medikament.

20. Verwendung einer Zusammensetzung gemäß einem der Ansprüche 1 bis 19 zur Herstellung eines Medikaments für die prophylaktische oder therapeutische Behandlung von bakteriellen Krankheiten.

21. Verfahren zur Herstellung einer Zusammensetzung gemäß einem der Ansprüche 1 bis 15, das Folgendes umfasst:
(i) in einem ersten Stadium die Herstellung einer Lösung A, die das Dispergieren des Expectorans in einem Teil des Eisessigs, die Zugabe des Stabilisators bzw. der Stabilisatoren und anschließend die Zugabe eines Teils des Wassers sowie das Mischen und Erhitzen, bis das Expectorans vollständig aufgelöst ist, umfasst;
(ii) in einem zweiten Stadium die Herstellung einer Lösung B, die das Auflösen des Antibiotikums des Chinolon- oder Naphthyridon-Typs oder seines Derivats im Rest des Eisessigs und des Wassers durch einfaches Mischen bei Raumtemperatur umfasst;
(iii) in einem dritten Stadium das Mischen von A und B und das Homogenisieren der erhaltenen Zusammensetzung.

22. Verfahren gemäß Anspruch 21, wobei der Teil des Eisessigs und des Wassers, der in Lösung A verwendet wird, jeweils etwa 40% der in der endgültigen Zusammensetzung vorhandenen Gesamtmenge dieser Komponenten ausmacht.

23. Verfahren zur Herstellung einer Zusammensetzung gemäß einem der Ansprüche 1 bis 15, das die folgenden Schritte umfasst: zuerst das Auflösen des Expectorans in der Gesamtmenge des Eisessigs, dann die Zugabe des Stabilisators bzw. der Stabilisatoren, die Zugabe von Wasser, das Mischen und Erhitzen, bis das Expectorans vollständig aufgelöst ist, und dann die Zugabe des Antibiotikums des Chinolon- oder Naphthyridon-Typs oder seines Derivats und das Mischen bis zur Auflösung, mit der Maßgabe, dass das nichtionische Tensid erst nach dem Antibiotikum zugegeben wird.

24. Verfahren gemäß einem der Ansprüche 20 bis 23, wobei die Temperatur, bis auf die die Lösung erhitzt wird, bis das Expectorans vollständig aufgelöst ist, zwischen etwa +50 °C und +60 °C liegt.

25. Verfahren gemäß Anspruch 24, wobei die Temperatur, bis auf die die Lösung erhitzt wird, bis das Expectorans vollständig aufgelöst ist, +60 °C beträgt.

26. Verfahren gemäß einem der Ansprüche 20 bis 25, das weiterhin den Schritt des Filtrierens der Zusammensetzung umfasst.

## Revendications

1. Une composition de formulation simple aqueuse comprenant un antibiotique de type quinolone ou de type naphtyridone, ou un dérivé chimiquement actif en dérivant choisi parmi les dérivés ester en C₁-C₄, un expectorant, un agent de stabilisation, de l'acide acétique glacial et de l'eau, l'agent stabilisant étant choisi parmi une combinaison d'agents tensioactifs non ioniques avec un ou plusieurs composants choisis parmi l'alcool benzylique, la N-méthylpyrrolidone, la 2-pyrrolidone, le diméthylacétamate (DMA), le diméthylformamide (DMF) et le diméthylsulfoxyde (DMSO), l'agent tensioactif non ionique présentant un HLB compris entre 10 et 18 et étant choisi dans le groupe consistant en les esters de polyalcool, les esters de macrogol, les esters de sorbitan, les polysorbates, les éthers de macrogol, les poloxamères et les alcools polyvinyliques ainsi que leurs mélanges.

2. Une composition selon la revendication 1, dans laquelle l'agent tensioactif non ionique est le Tween-80 ou le Cremophore.

3. Une composition selon la revendication 1 ou la revendication 2, dans laquelle l'expectorant est choisi parmi le chlorhydrate de bromhexine, l'ambroxol ou ses sels, la carbocystéine, le guaiacol ou ses sels de phosphate ou de benzoate, la guaifenesine, ou le guaiacolsulfonate de potassium.

4. Une composition selon la revendication 3, dans laquelle l'expectorant est le chlorhydrate de bromhexine.

5. Une composition selon l'une quelconque des revendications 1 à 4, dans laquelle l'antibiotique est choisi parmi l'enrofloxacine, la ciprofloxacine, la norfloxacine, la danofloxacine ou la sarafloxacine.

6. Une composition selon l'une quelconque des revendications 1 à 5, dans laquelle la quantité d'agent antibiotique est comprise entre 5 % et 40 % en poids/volume.

7. Une composition selon la revendication 6, dans laquelle la quantité d'antibiotique est de 20 % en poids/volume.

8. Une composition selon l'une quelconque des revendications 1 à 7, dans laquelle la quantité d'expectorant est comprise entre 0,1 % et 4 % en poids/volume.

9. Une composition selon la revendication 8, dans laquelle la quantité d'expectorant est de 1 % en poids/volume.

10. Une composition selon l'une quelconque des revendications 1 à 9, dans laquelle la quantité d'acide acétique est comprise entre 5 % et 16 % en poids/volume.

11. Une composition selon la revendication 10, dans laquelle la quantité d'acide acétique est de 8 % en poids/volume.

12. Une composition selon l'une quelconque des revendications 1 à 11, dans laquelle la quantité d'eau est supérieure ou égale à 35 % en poids/volume.

13. Une composition selon l'une quelconque des revendications 1 à 12, dans laquelle l'agent stabilisant est une combinaison choisi dans le groupe consistant en Tween-80 et 2-pyrrolidone, Tween-80 et N-méthylpyrrolidone, Tween-80 et diméthylacétamate (DMA), Tween-80 et diméthylformamide (DMF), Tween-80 et diméthylsulfoxyde (DMSO), ou Tween-80, 2-pyrrolidone et alcool benzylique.

14. Une composition selon l'une quelconque des revendications 1 à 13, dans laquelle la quantité d'agent stabilisant est comprise entre 9 % et 30 % en poids/volume.

15. Une composition selon l'une quelconque des revendications 1 à 14, qui comprend 20 % en poids/volume d'enrofloxacine, 1 % en poids/volume de chlorhydrate de bromhexine, 4 % en poids/volume de 2-pyrrolidone et 10 % en poids/volume de Tween-80.

16. Une composition selon l'une quelconque des revendications 1 à 15, comprenant en outre de la formaline.

17. Une composition selon la revendication 16, dans laquelle la quantité de formaline est comprise entre 0,1 % et 3 % en poids/volume.

18. Une composition selon l'une quelconque des revendications 1 à 17, comprenant en outre des excipients, des conservateurs, des vitamines, des supports, des antioxydants, des agents de photostabilisation, des colorants ou des épaississants.

19. Une composition selon l'une quelconque des revendications 1 à 18, pour son utilisation en tant que médicament.

20. Utilisation d'une composition selon l'une quelconque des revendications 1 à 19 pour la préparation d'un médicament pour le traitement prophylactique ou thérapeutique des maladies bactériennes.

21. Procédé pour la préparation d'une composition selon l'une quelconque des revendications 1 à 15 comprenant :
(i) dans une première étape, la préparation d'une solution A comprenant la dispersion de l'expectorant dans une partie de l'acide acétique glacial, l'addition d'agent(s) stabilisant(s) suivie de l'addition d'une partie de l'eau, et le mélange et le chauffage jusqu'à ce que l'expectorant soit complètement dissous ;
(ii) dans une seconde étape, la préparation d'une solution B comprenant la dissolution de l'antibiotique de type quinolone ou naphtyridone ou ses dérivés dans le reste de l'acide acétique glacial et de l'eau par simple mélange à la température ambiante ;
(iii) dans une troisième étape, le mélange de A et B et l'homogénéisation de la composition obtenue.

22. Procédé selon la revendication 21, dans lequel la partie de l'acide acétique glacial et de l'eau utilisés dans la solution A représentent environ 40 % de la quantité totale de chacun de ces composants présents dans la composition finale.

23. Procédé de préparation d'une composition selon l'une quelconque des revendications 1 à 15, comprenant les étapes de premièrement dissoudre l'expectorant dans la totalité de l'acide acétique glacial, suivi de l'addition du ou des agents de stabilisation, l'addition d'eau, le mélange et le chauffage jusqu'à ce que l'expectorant soit complètement dissous, et ensuite l'addition de l'antibiotique de type naphtyridone ou quinolone ou un de ses dérivés, et le mélange jusqu'à dissolution avec cette condition que l'agent tensioactif non ionique n'est ajouté qu'après l'antibiotique.

24. Procédé selon l'une quelconque des revendications 20 à 23, dans lequel la température jusqu'à laquelle la solution est chauffée jusqu'à ce que l'expectorant soit complètement dissous est comprise entre +50°C et +60°C.

25. Procédé selon la revendication 24, dans lequel la température jusqu'à laquelle la solution est chauffée jusqu'à ce que l'expectorant soit complètement dissous est de +60°C.

26. Procédé selon l'une quelconque des revendications 20 à 25, comprenant en outre une étape de filtration de la composition.
